# EUROPEAN PATENT SPECIFICATION

(11) **EP 0 886 522 B1**
(45) Date of publication and mention of the grant of the patent: **31.07.2002**
(21) Application number: 97902204.3
(22) Date of filing: 23.01.1997
(51) Int. Cl.: A61K 35/20, A61P 1/10

(54) **USE OF COLOSTRUM FOR THE TREATMENT OF CONSTIPATION**
VERWENDUNG VON KOLOSTRUM ZUR BEHANDLUNG VON DARMVERSTOPFUNG
UTILISATION DU COLOSTRUM DANS LE TRAITEMENT DE LA CONSTIPATION

(30) Priority: 24.01.1996 IT MI960113
(43) Date of publication of application: 30.12.1998
(73) Proprietor: Pharmaproducts UK Limited, Liverpool, Merseyside L2 9TL (GB)
(72) Inventor: FERRARIO, Gianluigi, Tortola (VG)
(74) Representative: Bianchetti, Giuseppe, Prof.
(86) International application number: EP9700300
(87) International publication number: WO9726899

(56) References cited:
- EP-A- 0 652 013
- WO-A-94/16675
- FR-A- 2 622 109

## Description

The present invention relates to the use of colostrum for the treatment of constipation.

Constipation is a widespread problem in adults and children, consisting in the difficult or infrequent passage of the stool mass through the colon, which mass accumulates becoming hard following water reasdorption.

Constipation can be acute or chronic, due to organic, traumatic, functional, neurogenic conditions and the like.

The treatment of constipation involves various alternatives:
- Enrichment in the vegetable fibres content of the diet, use of agents increasing stool bulk (natural or semisynthetic polysaccharides and cellulose derivatives, such as bran, psyllium and methylcellulose) or mineral oils, to increase stool bulk and keep it softer, thanks to an increased adsorption of liquids in the intestinal tract. Such an approach, however, is not always sufficient to overcome the problem.
- Use of saline or osmotic laxatives, such as salts (magnesium, sodium and potassium sulfates, phosphates and tartrates); lactulose, sorbitol: they transit rapidly in the bowel, where they cause an increase in osmotic pressure and therefore an increase in water adsorption in the feces and the peristalsis stimulation.

This laxatives act rapidly and are generally used to empty the bowel prior to diagnostic procedures and surgery; they are contraindicated in case of renal failure and heart failure.
- Use of stimulant laxatives (phenolphthalein, castor oil, anthraquinone derivatives such as senna and cascara), which promote an increase in peristalsis and intraluminal fluid. They can cause a remarkable irritation of bowel and electrolyte balance disturbances.

Therefore, a medicament promoting elimination without causing the above mentioned side-effects is much needed.

The present invention aims at overcoming the above cited problems, by the use of colostrum for the preparation of a medicament for the treatment of constipation.

The present invention further relates to the use of colostrum, as a laxative.

It has surprisingly been found that colostrum, administered to patients suffering from constipation, acts favourably inducing elimination of stool mass without causing irritation, cramps or electrolyte disturbances.

In fact, during the clinic trials, 11 out of 12 treated patients showed a favourable response to the treatment with colostrum, obtaining an effective, natural laxative action.

It should be stressed that colostrum exerts and effective action in the treatment of women suffering from pregnancy or post-partum constipation, which are delicate conditions difficult to treat, as the response to the treatment with both bulking agents and common laxatives is poor, and contraindications to pharmacological treatment are remarkable, since drugs can induce uterus contractions in the pregnant woman (as is the case with castor oil) or they can be present in breast milk in amounts adversely affecting the breastfed infant (particularly anthraquinone laxatives). The colostrum treated patients obtained an effective, physiological laxative effect without any side-effects.

The gastro-resistant pharmaceutical formulations contain powder or liquid colostrum in amounts of 10 mg to 5 g per unitary dose.

More particularly, the formulations comprise gastro-resistant tablets containing powdered, freeze-dried or sprayzed colostrum, or gastro-resistant pearls containing liquid colostrum.

The gastro-resistant tablets or pearls are prepared according to the conventional pharmaceutical techniques for the preparation of gastro-resistant formulations, for example an enteric coating can be applied to a colostrum core by means of fluidized bed coating or pan coating techniques.

The enteric coating is used to protect colostrum against the gastric acid environment, such a coating can consist of copolymerized methacrylic acid/methacrylic acid methyl esters, such as those known under the names Eudragit^{R} (commercialized by Rohm Pharma), polymers such as cellulose acetate phthalate, hydroxypropyl methylcellulose phthalate, water-based polymer dispersions, such as Eudragit^{R} (by Rohm Pharma), Aquateric^{R} (FMC Corp.). The enteric coating can optionally contain plasticizers such as triacetin, cetanol, citric and phthalic acids esters.

The formulations can optionally contain other active ingredients known to have a beneficial effect on the condition to treat, particularly vegetable extracts. According to a further aspect, the formulations can contain colostrum mixed with milk.

The following examples further illustrate the invention.

### Example 1

One enteric-coated tablet contains:

| | |
|---|---|
| Powder colostrum | 145 mg |
| Hydroxypropyl methylcellulose phthalate | 6 mg |
| Cetyl alcohol | 0.5 mg |

The enteric coating solution is applied on the colostrum core by fluidized bed coating.

### Example 2

One enteric-coated tablet contains:

| | |
|---|---|
| Powder colostrum | 64 mg |
| Powder milk | 45 mg |
| Hydroxypropyl methylcellulose phthalate | 32 mg |
| Cetyl alcohol | 5 mg |

The enteric coating solution is applied on the colostrum core by fluidized bed coating.

### Example 3

One 400 mg capsule contains:

| | |
|---|---|
| Freeze-dried colostrum | 12 mg |
| Cassia acutifolia dried leaves | 25 mg |
| Aloe vera | 40 mg |
| Gelidum e Gracilaria | 120 mg |
| Rheum Rhaponticum rhizome | 85 mg |
| Carbo ligni | 118 mg |

The whole mass is mixed thoroughly, then placed into a gastro-resistant capsule.

### Example 4

One 400 mg capsule contains:

| | |
|---|---|
| Freeze-dried colostrum | 60 mg |
| Cassia acutifolia dried leaves | 25 mg |
| Aloe vera | 40 mg |
| Gelidum e Gracilaria | 105 mg |
| Rheum Rhaponticum rhizome | 80 mg |
| Carbo ligni | 90 mg |

The whole mass is mixed thoroughly, then placed into a gastro-resistant capsule.

## Claims

1. The use of colostrum for the preparation of a medicament for the treatment of constipation.

## Patentansprüche

1. Verwendung vom Kolostrum zur Herstellung eines Arzneimittels zur Behandlung der Obstipation.

## Revendications

1. Utilisation du colostrum pour la préparation d'un médicament destiné au traitement de la constipation.
